# EUROPÄISCHE PATENTSCHRIFT

(11) **EP 1 075 294 B1**
(45) Veröffentlichungstag und Bekanntmachung des Hinweises auf die Patenterteilung: **29.09.2004**
(21) Anmeldenummer: 99932616.8
(22) Anmeldetag: 30.04.1999
(51) Int. Cl.: A61M 15/08

(54) **VORRICHTUNG ZUM EINSETZEN IN DIE MENSCHLICHE NASE**
DEVICE FOR INSERTING INTO THE HUMAN NOSE
DISPOSITIF A INTRODUIRE DANS LE NEZ D'UN ETRE HUMAIN

(30) Priorität: 30.04.1998 DE 29807851 U; 17.07.1998 DE 19832205
(43) Veröffentlichungstag der Anmeldung: 14.02.2001
(73) Patentinhaber: Hexal AG, 83607 Holzkirchen (DE)
(72) Erfinder: KAROW, Eva-Maria, D-65604 Elz (DE); STIRMANN, Jonathan, D-55546 Frei-Laubersheim (DE)
(74) Vertreter: Philipp, Matthias, Dr.
(86) Internationale Anmeldenummer: PCT/DE1999/001332
(87) Internationale Veröffentlichungsnummer: WO 1999/055404

(56) Entgegenhaltungen:
- EP-A- 0 780 137
- WO-A-88/03788
- FR-A- 407 679
- FR-A- 1 001 434
- FR-A- 2 419 711

## Beschreibung

Die Erfindung betrifft eine Vorrichtung zum Einsetzen in die menschliche Nase, die sich insbesondere zur Applikation von pharmazeutischen Wirkstoffen und zur Exposition von Diagnostika in der Nase und zur Verbesserung der Nasenatmung eignet.

### Hintergrund für die Verbesserung der Nasenatmung

Die Ursachen für die Behinderung der Nasenatmung sind vielfältig. So können angeborene und erworbene Deformitäten der knöchernen bzw. knorpeligen Nasenstruktur die Ursache darstellen wie auch chronisch-entzündliche oder aber degenerative Gewebsveränderungen.

Der Atemstrom durch den menschlichen Körper beginnt mit dem Fluß durch den Nasenvorhof, das sog. vestibulum nasi. Deformitäten der Nasenknorpel und mangelnde Elastizität der bindegeweblichen Strukturen können zu eine Einengung des vestibulum nasi führen. Darüber hinaus kann es bei der Einatmung zu einem Ansaugen der Nasenflügel nach innen mit konsekutiver Nasenatmungsbehinderung kommen. Da für letzteres häufig degenerative Veränderungen der Bindegewebsstrukturen verantwortlich sind, findet sich dieses Phänomen gehäuft im fortgeschrittenen Lebensalter und bedingt nicht selten lästige Schnarchgeräusche.

Bei der Anwendung zur Verbesserung der Nasenatmung wird eine Optimierung der Lumenverhältnisse im Bereich des vestibulum nasi bewirkt. Dazu wird die ggf. schmetterlingsförmige Vorrichtung im Bereich des vestibulum nasi derart plaziert, daß sie die Nasenflügel weit aufspannt bzw. ein Einsinken der Nasenflügel während der Inspiration verhindert und somit den Innendurchmesser des vestibulum nasi fixiert bzw. vergrößert.

### Hintergrund für die Applikation von pharmazeutischen Wirkstoffen

Die Nasenschleimhaut verfügt über gute resorptive Eigenschaften und eignet sich daher prinzipiell gut für eine alternative Applikation für pharmazeutische Wirkstoffe. Es existieren Versuche, sowohl Impfstoffe als auch unterschiedliche pharmazeutische Wirkstoffe über die Nasenschleimhaut zu applizieren. Als problematisch in diesem Zusammenhang hat sich der natürliche Clearance-Apparat der Nasenhaupthöhle herausgestellt. So ist dieser Apparat in der Lage, auf die Nasenschleimhaut applizierte Stoffe durchschnittlich innerhalb von 10 Minuten zu klären, d.h. rachenwärts zu transportieren, von wo aus diese Stoffe dann entweder expektoriert oder geschluckt werden, was eine ausreichende Resorption verhindert.

Herkömmlicherweise werden zur Applikation von pharmazeutischen Wirkstoffen in der Nasenhaupthöhle Darreichungsformen wie Lösungen oder Salben gewählt. Diese Formen haben den großen Nachteil, daß sie den pharmazeutischen Wirkstoff in einer Art Bolus applizieren. Das führt zum einen dazu, daß dieser Bolus schon recht schnell durch die normale Clearanceleistung der Nasenschleimhaut abtransportiert wird. Zum anderen wird durch die kurzzeitige massive Belastung der Nasenschleimhaut die Reinigungsleistung des Clearance-Apparates angeregt, so daß die Abtransportrate noch erhöht wird. Das führt dazu, daß die applizierten pharmazeutischen Wirkstoffe häufig schneller per mukoziliärer Clearance eliminiert werden, als sie resorbiert werden können.

### Hintergrund für die Applikation von Diagnostika

Durch ihre Nähe und ihren ständigen Kontakt mit der Umwelt ist die Nasenschleimhaut in besonderem Maße äußeren Reizen ausgesetzt, die nicht selten zu Überempfindlichkeitsreaktionen bis hin zu manifesten Allergien führen. Der Klärung des auslösenden Agens kommt dabei große Bedeutung zu, da nur dadurch eine ursächliche Behandlung in Form von Allergenkarenz oder Hyposensibilisierung vorgenommen werden kann. Bislang werden dazu sogenannte Intrakutanteste bzw. Prickteste eingesetzt. Es handelt sich dabei um in vivo Testverfahren, die die Reaktivität des Körpers auf bestimmte Antigene in der Haut des Unterarms oder des Rückens testen. Ihr großer Nachteil ist, daß diese Testverfahren keine Aussage über die klinische Relevanz ihrer Ergebnisse zulassen, da sie keine Auskunft über das akute Geschehen in der Nase geben. Man nutzt diese Untersuchungen daher häufig als Screening-Untersuchung, um dann anschließend gezielter eine nasale Provokation durchzuführen. Diese sind sehr sensitiv und spezifisch, haben aber den Nachteil, daß sie zu starken lokalen Reaktionen beim Patienten führen können und die Testung verschiedener Allergene nicht unmittelbar hintereinander erfolgen kann.

Beispielsweise kommt es im Rahmen einer Rhinitis allergica zu einer Ausbildung spezifischer Antikörper gegen das Antigen, die zumindest zu Beginn der Erkrankung vorwiegend lokal abläuft. Antikörper sind daher besonders in der Nasenschleimhaut und im Nasensekret zu finden.

Aus der FR-A-2 419 711 ist eine Vorrichtung nach dem Oberbegriff des Anspruchs 1 bekannt.

Die Aufgabe der Erfindung besteht darin, eine Vorrichtung zum Einsetzen in die menschliche Nase bereitzustellen, die sich einerseits zur Verbesserung der Nasenatmung eignet, d.h. hinsichtlich der Anpaßbarkeit an unterschiedliche Abmessungen des Nasenvorhofes Vorteile bietet und auch nach Möglichkeit eine größere freie Querschnittsfläche für den Atemluftstrom erzeugt, und die andererseits auch einen Träger bzw. ein Gerüst für therapeutische Systeme bildet, mit dem pharmazeutischen Wirkstoff auf die Nasenschleimhaut appliziert werden können, um diese dann verzögert freigeben zu können, oder aber der mit Diagnostika versehen ist, um unterschiedliche Diagnosereaktionen bzw. Tests in einfacher Weise durchzuführen zu können.

Diese Aufgabe wird erfindungsgemäß durch eine Vorrichtung nach Anspruch 1 gelöst.

Das Grundelement kann leicht schmetterlingsförmig mit zwei zu einer Mittellinie symmetrisch angeordneten Flügeln ausgebildet sein.

Das Grundelement kann seitlich mit Anschrägungen versehen sein. Das Grundelement kann in seinem mittleren Bereich eine Verdickung aufweisen.

Es kann vorgesehen sein, daß das Grundelement einen faden- oder stangenartigen Fortsatz zum Einsetzen und Entfernen aufweist.

Die Vorrichtung kann aus einem Material bestehen, das geeignet ist, über längere Zeit in der Körperhöhle zu verbleiben, beispielsweise aus Silikonkautschuk.

In einer bevorzugten Ausführungsform kann vorgesehen sein, daß das Grundelement auf der Innenseite und/oder der Außenseite mit mindestens einem zu applizierenden pharmazeutischen Wirkstoff und/oder mindestens einem Diagnostikum versehen ist.

In einer Ausführungsform der Vorrichtung ist vorgesehen, daß auf der Innenseite und/oder auf der Außenseite Mulden angeordnet sind.

Es kann vorgesehen sein, daß auf der Innenseite und/oder Außenseite, ggf. in den Mulden Plättchen oder Schwämme auf Cellulosebasis als Träger für pharmazeutische Wirkstoffe und/oder Diagnostika angeordnet sind. Alternativ können auf der Innenseite und/oder Außenseite, ggf. in den Mulden, auch transmukosale und/oder nasale therapeutische Reservoir- oder Matrixsysteme als Träger für pharmazeutische Wirkstoffe und/oder Diagnostika angeordnet sein.

In einer bevorzugten Ausführungsform kann vorgesehen sein, daß ein zweites Grundelement unter Bildung einer Einheit über ein Verbindungsmittel mit dem Grundelement verbunden ist.

Die Erfindung ermöglicht die Verwendung der erfindungsgemäßen Vorrichtung zur Verbesserung der Nasenatmung und/oder zur Applikation von pharmazeutischen Wirkstoffen und/oder zur Exposition von Diagnostika in der Nase.

Bei der Anwendung zur Applikation von pharmazeutischen Wirkstoffen können diese in einer Trägerschicht bzw. Matrix ein- oder beidseitig auf die Vorrichtung aufgebracht werden und ihre Freigabe entweder direkt aus der Matrix oder durch eine darüber angeordnete Diffusionsschicht (Membran) beliebig eingestellt werden. Dadurch wird erreicht, daß die pharmazeutischen Wirkstoffe gleichmäßig in geringen Dosen abgegeben werden, was ihre Resorptionsrate insgesamt erhöht. Zusätzlich können durch die beliebig lange Anwendung gleichmäßige Wirkspiegel über einen definierten Zeitraum erreicht werden.

Bei der Anwendung zur Exposition von Diagnostika können die entsprechenden Substanzen, die fest an die erfindungsgemäße Vorrichtung gebunden sind und mit der Umgebung kommunizieren können, über einen Zeitraum von z.B. ca. 20-30 Minuten im Nasenvorhof plaziert werden. Zum Beispiel kann es bei Vorliegen einer Allergie auf ein so stationiertes Antigen zu einer Antigen-Antikörper-Reaktion kommen, die später mittels enzymatischer oder radiologischer Immuntests nachgewiesen werden kann. Mit dieser Methode kann somit eine Aussage über das aktuelle Geschehen in der Nasenschleimhaut getroffen werden, ohne daß dabei der Patient mit invasiven oder gesundheitsstörenden Maßnahmen belastet wird. Zudem ist mit der Methode die Testung verschiedener Allergene gleichzeitig möglich, denn auf der erfindungsgemäßen Vorrichtung können ca. 10 oder mehr Antigene aufgebracht werden, was eine schnelle und überall durchführbare Diagnostik, auch im Praxisalltag, erleichtert. Die Erfindung ist selbstverständlich aber nicht nur auf die Exposition von Allergenen beschränkt; alle Diagnostika, die im Bereich der Nase sinnvoll eingesetzt werden können, sind einsetzbar.

Weitere Vorteile und Merkmale der Erfindung ergeben sich aus der nachfolgenden Beschreibung bevorzugter Ausführungsbeispiels, wobei auf eine Zeichnung Bezug genommen wird, in der
Fig. 1 eine Draufsicht auf die Vorderseite der erfindungsgemäßen Vorrichtung in einer ersten Ausführungsform zeigt,
Fig. 2 eine Draufsicht auf die Rückseite der erfindungsgemäßen Vorrichtung gemäß Fig. 1 zeigt,
Fig. 3(a) bis (c) Ansichten von oben in drei unterschiedlichen Biegestellungen zeigen,
Fig. 4 die Fixierungsposition der Vorrichtung im Nasenvorhof zeigt,
Fig. 5 die Aufbringung eines pharmazeutischen Wirkstoffs und einer Diffusionsschicht (Membran) auf die erfindungsgemäße Vorrichtung zeigt,
Fig. 6 die Aufbringung von Diagnostika/pharmazeutischen Wirkstoffen auf der erfindungsgemäßen Vorrichtung zeigt,
Fig. 7 eine weitere Ausführungsform der erfindungsgemäßen Vorrichtung zeigt,
Fig. 8 die Vorrichtung gemäß Fig. 7 in der Fixierungsposition im Nasenvorhof zeigt, und
Fig. 9 eine Variante der erfindungsgemäßen Vorrichtung zeigt.

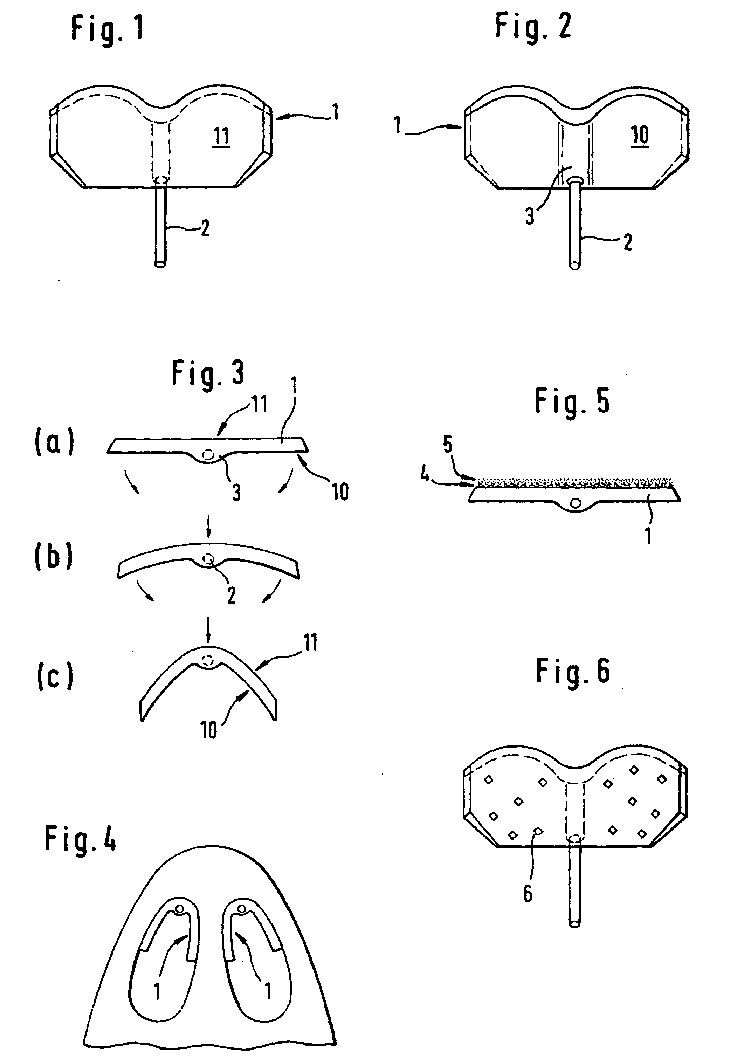

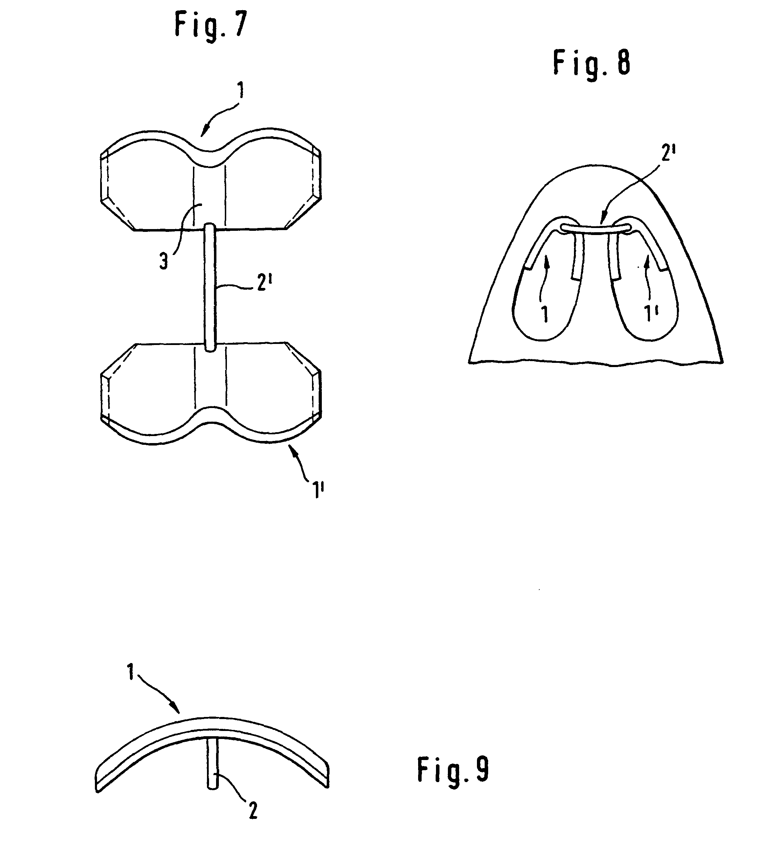

Fig. 1 bis 6 zeigen Aufbau und Wirkungsweise der erfindungsgemäßen Vorrichtung, wobei die Vorrichtung einzeln verwendet werden kann, d.h. jeweils für das linke oder das rechte Nasenloch, wie Fig. 4 zeigt, wobei die beiden Vorrichtungen identisch sind, oder auch als Einheit aus zwei Grundelementen 1 und 1', bei der die Grundelemente der beiden Vorrichtungen über ein bspw. fadenförmiges Verbindungsmittel 2' miteinander verbunden sind (Fig. 7, 8).

Fig. 1 und 2 zeigen jeweils eine (ebene) Draufsicht von vom und von hinten auf die im wesentlichen plattenförmige Vorrichtung, wobei aus der Ansicht von oben nach Fig. 3(a) die im wesentlichen konstante Dicke der Vorrichtung hervorgeht. Das plattenförmige Grundelement 1 ist im wesentlichen rechteckförmig bzw. bevorzugt leicht schmetterlingsförmig, wie Fig. 1 und 2 zeigen, und ist in Form und Größe den Verhältnissen des Nasenvorhofs (vestibulum nasi) so angepaßt, daß es darin fest plaziert werden kann. Das Grundelement 1, das im unbelasteten Zustand im wesentlichen eben oder leicht gebogen ist und damit durch seine elastische Verformbarkeit eine normalerweise für ein gutes Anliegen ausreichendes Spreizkraft aufweist, wird durch einfaches Einlegen in den Nasenvorhof (Fig. 4) fest plaziert und kann grundsätzlich beliebige Zeit dort verbleiben. Dicke und/oder Größe (Breite und Länge) des Grundelements sind den individuellen Bedürfnissen des Benutzers angepaßt, indem z.B. abgestufte Maße bereitgestellt werden. Zur Verbesserung der Plazierung können (auch) an den seitlichen Rändern der Vorrichtung Verdickungen vorgesehen sein, entsprechend der mittleren Verdickung 3.

Die sichere Fixierung wird neben der besonderen Konstruktion (Form der Flügel, Abschrägungen nach hinten, Giebelform etc.), die den anatomischen Verhältnissen angepaßt ist, vor allem auch durch die konstruktiv bzw. durch geeignete Materialauswahl bedingte Spreizkraft erreicht. Die erfindungsgemäße Vorrichtung liegt somit an der Schleimhaut des Nasenvorhofs fest an und steht mit dieser über die Außenfläche 11 des Grundelements in direktem Kontakt, während die Innenfläche 10 des Grundelements mit dem Atemluftstrom in Kontakt steht.

Zur Erleichterung des Einsetzens und Herausnehmens der Vorrichtung ist der plattenförmige Grundkörper 1 mit einem faden- oder stangenartigen Fortsatz 2 versehen, der sich von der unteren Längskante des Grundelements erstreckt. Weiterhin ist das plattenförmige Grundelement an seinem mittleren Bereich mit einer Verdickung 3 versehen, um Plazierung und Spreizkraft zu variieren bzw. einzustellen und zu verbessern. Alternativ kann auf eine derartige Verdickung verzichtet werden (Fig. 9), wenn durch das gewählte Material bzw. die Beschichtung mit einem den pharmazeutischen Wirkstoff enthaltenden mukosalen und/oder nasalen therapeutischen System oder mit Diagnostika die Spreizkraft für optimale Plazierung bzw. Befestigung gegeben ist.

Fig. 3(a) bis (c) zeigen unterschiedliche Stadien der Verformung der erfindungsgemäßen Vorrichtung zwecks Einsetzens in den Nasenvorhof, wobei dieser (End-)Zustand in Fig. 4 dargestellt ist. Die Vorrichtung wird mit ihrem medialen Teil zur Nasenspitze weisend eingeführt, wobei die Flügel an der lateralen und der medialen Nasenwand anliegen. Es sind keinerlei äußere Spangen, Verklebungen o.ä. erforderlich.

Die erfindungsgemäße Vorrichtung wird vorzugsweise aus Materialien hergestellt, die vorübergehend, d.h. bis zu etwa einem Monat (oder auch länger), in einer Körperhöhle wie der Nase verbleiben können und sich während dieser Zeit nicht auflösen sowie keine unerwünschten Nebenwirkungen hervorrufen, wie beispielsweise Silikonkautschuk.

Der (Die) zu applizierende(n) pharmazeutische(n) Wirkstoff(e) kann in eine Matrix eingebettet sein, die auf der Innen- und/oder Außenseite des Grundelementes aufgebracht ist. Für die Matrix kommen alle üblichen Matrixbildner für den medizinischen Einsatz in Frage, wie z.B. Polyacrylat, Silikon, Silikonöl, Polyisobutylen, Kautschuk, kautschukähnliche synthetische Homo-, Co- oder Blockpolymere, Butylkautschuk, Styrol/Isopren-Copolymere, Polyurethane, Copolymere des Ethylens, Polysiloxane oder Styrol/-Copolymere.

Das transmukosale und/oder nasale therapeutische System zur Applikation von pharmazeutischen Wirkstoffen, welches auf die erfindungsgemäße Vorrichtung aufgebracht ist, kann ausschließlich aus der eben beschriebenen Matrix bestehen, die den (die) pharmazeutischen Wirkstoff(e) enthält. Die verzögerte Freisetzung der pharmazeutischen Wirkstoffe erfolgt dabei direkt aus der Struktur der Matrix heraus.

Eine andere Möglichkeit (wie dargestellt in Fig. 5) besteht darin, über der den (die) pharmazeutischen Wirkstoff(e) enthaltenden Schicht 4 (die auch eine wie zuvor beschriebene Matrix darstellen kann) eine Diffusionsschicht (5), auch Membran genannt, für die Kontrolle der Freisetzung der pharmazeutischen Wirkstoffe aufzubringen. Eine solche Diffusionsschicht besteht beispielsweise aus inerten Polymeren, insbesondere auf der Basis von Polypropylen, Polyvinylacetat, Polyamid, Ethylen-Vinylacetat oder Silikon.

Als pharmazeutische Wirkstoffe kommen u.a. Hormone (Calcitonin, Insulin, Heparin) und Impfstoffe (aktive Immunisierung) in Betracht. Weitere Beispiele sind Vasokonstriktoren oder NO-Synthase-Hemmer, Nitroglycerin, ätherische Öle, Analgetika, z.B. Morphium und dessen Derivate, Migränemittel, z.B. Triptane (Sumatriptan, Rizatriptanbenzoat, Naratriptan) oder auch Ergotaminderivate, und Scopolamin.

Im Unterschied zu pharmazeutischen Wirkstoffen bleiben Diagnostika fest an oder in der Vorrichtung fixiert und werden nach Ablauf der Untersuchungszeit (z.B. 30 Minuten) analysiert. Als Beispiele für Diagnostika seien Allergene, wie etwa Schimmelpilzallergene, Milbenkotallergene und alle Arten von Pollenallergenen, sowie Bindungsproteine für Zytokine und Zellmarker genannt.

Zur Erleichterung des Einsetzens und Herausnehmens der Vorrichtung ist das plattenartige Grundelement normalerweise mit einem faden- oder stangenartigen Fortsatz versehen, der sich von einer Längskante des Grundelements erstreckt. Eine weitere Möglichkeit besteht darin, zwei Grundelemente miteinander zu verbinden, z.B. über den genannten Fortsatz. Eine derartige Verbindung könnte bspw. die Form eines dünnen Fadens bzw. einer Schnur haben, beispielsweise aus Silikonkautschuk. Dadurch ist die gleichzeitige Anwendung zweier Grundelemente, d.h. für jedes Nasenloch eine, ohne die Gefahr der Aspiration oder des Verschleppens in den hinteren Bereich der Nebenhaupthöhle möglich. Außerdem wird das Einsetzen und Herausnehmen weiter erleichtert. Fig. 7 und 8 erläutern diese Ausgestaltung.

Weiter kann vorgesehen sein, daß sich auf der Innenseite 10 und/oder der Außenseite 11 der Vorrichtung Mulden 6 angeordnet sind (Fig. 6), in die beispielsweise Plättchen oder Schwämme auf Cellulosebasis als Träger bspw. für chemisch gebundene Allergene eingebracht werden können. Alternativ können derartige allergenhaltige Plättchen auf Cellulosebasis auch unmittelbar auf die Vorrichtung aufgebracht werden, ohne daß eine Mulde vorhanden sein muß. Auch besteht die Möglichkeit, übliche transmukosale und/oder nasale therapeutische Reservoir- bzw. Matrixsysteme (mit oder ohne Diffusionsschicht), welche einen oder mehrere pharmazeutische Wirkstoff(e) enthalten, in die Mulden einzufügen. Pharmazeutische Wirkstoffe, die transmukosal wirken sollen, können auf der der Nasenschleimhaut zugewandten Seite (Außenseite 11) integriert werden. Inhalativ wirkende Stoffe, beispielsweise ätherische Öle, können auf der der Schleimhaut abgewandten bzw. dem Atemluftstrom zugewandten Seite (Innenseite 10) eingebracht werden.

Die Erfindung bietet somit die Vorteile, (1) gut, leicht und sicher im Nasenvorhof zu plazieren und wieder zu entfernen zu sein, (2) in gleichmäßigem und gutem Kontakt mit der Schleimhaut des Nasenvorhofs zu stehen, und (3) hinsichtlich der Applikationsdauer beliebig variabel gestaltbar zu sein.

Die in der vorangehenden Beschreibung, in der Zeichnung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Vorrichtung zum Einsetzen in die menschliche Nase, mit einem elastisch verformbaren, plattenartigen Grundelement (1, 1'), das entlang einer Mittellinie U-förmig verformbar und im U-förmig verformten Zustand zwischen Nasenscheidewand und Nasenflügel einsetzbar ist, wobei das Grundelement (1, 1') eine einerseits auf die Nasenscheidewand und andererseits auf den Nasenflügel gerichtete Spreizkraft ausüben kann, **dadurch gekennzeichnet, dass** das Grundelement (1,1') im unbelasteten, nicht in die Nase eingesetzten Zustand eben oder leicht gebogen ist.

2. Vorrichtung nach Anspruch 1, **dadurch gekennzeichnet, daß** das Grundelement leicht schmetterlingsförmig mit zwei zu einer Mittellinie symmetrisch angeordneten Flügeln ausgebildet ist.

3. Vorrichtung nach Anspruch 1 oder 2, **dadurch gekennzeichnet, dass** das Grundelement seitlich mit Anschrägungen versehen ist.

4. Vorrichtung nach einem der vorangehenden Ansprüche, **dadurch gekennzeichnet, daß** das Grundelement in seinem mittleren Bereich eine Verdickung (3) aufweist.

5. Vorrichtung nach einem der vorangehenden Ansprüche, **dadurch gekennzeichnet, daß** das Grundelement einen faden- oder stangenartigen Fortsatz (2) zum Einsetzen und Entfernen aufweist.

6. Vorrichtung nach einem der vorangehenden Ansprüche, **dadurch gekennzeichnet, daß** sie aus einem Material besteht, das geeignet ist, über längere Zeit in einer Körperhöhle zu verbleiben.

7. Vorrichtung nach Anspruch 6, **dadurch gekennzeichnet, daß** sie aus Silikonkautschuk besteht.

8. Vorrichtung nach einem der vorangehenden Ansprüche, **dadurch gekennzeichnet, daß** das Grundelement (1, 1') mit mindestens einem zu applizierenden pharmazeutischen Wirkstoff und/oder mindestens einem Diagnostikum versehen ist.

9. Vorrichtung nach einem der vorangehenden Ansprüche, **gekennzeichnet durch** Mulden (6).

10. Vorrichtung nach einem der Ansprüche 8 oder 9, **gekennzeichnet durch** ggf. in den Mulden (6) angeordnete Plättchen oder Schwämme auf Cellulosebasis als Träger für pharmazeutische Wirkstoffe und/oder Diagnostika.

11. Vorrichtung nach einem der vorangehenden Ansprüche 8 oder 9, **gekennzeichnet durch** ggf. in den Mulden (6) angeordnete transmukosale und/oder nasale therapeutische Reservoir oder Matrixsysteme als Träger für pharmazeutische Wirkstoffe und/oder Diagnostika.

12. Vorrichtung nach einem der vorangehenden Ansprüche, **dadurch gekennzeichnet, daß** ein zweites Grundelement (1') unter Bildung einer Einheit über ein Verbindungsmittel (12) mit dem Grundelement (1) verbunden ist.

13. Verwendung der Vorrichtung nach einem der vorangehenden Ansprüche zur Verbesserung der Nasenatmung und/oder zur Applikation von pharmazeutischen Wirkstoffen und/oder zur Exposition von Diagnostika in der Nase.

## Claims

1. A device for insertion into the human nose, with an elastically deformable base element (1, 1') in the form of a plate which is deformable in the shape of a U along a centre line and is insertable in the U-shaped state of deformation, between the septum and the side of the nose, the base element (1, 1') being able to exert an expanding force directed, on the one hand, towards the septum and, on the other hand, to the nose side, **characterised in that** the base element (1, 1') in the unloaded state when it is not inserted in the nose is flat or slightly bent.

2. A device according to claim 1, **characterised in that** the base element is slightly butterfly shaped with two wings disposed symmetrically in relation to the centre line.

3. A device according to claim 1 or 2, **characterised in that** the base element is provided with chamfers laterally.

4. A device according to any one of the preceding claims, **characterised in that** the base element has a thickening (3) in its middle zone.

5. A device according to any one of the preceding claims, **characterised in that** the base element has a continuation (2) in the form of a filament or rod for insertion and removal.

6. A device according to any one of the preceding claims, **characterised in that** it consists of a material suitable for remaining in a body cavity over a relatively long period.

7. A device according to claim 6, **characterised in that** it consists of silicone rubber.

8. A device according to any one of the preceding claims, **characterised in that** the base element (1, 1') is provided with at least one pharmaceutical active substance for application and/or at least one diagnostic agent.

9. A device according to any one of the preceding claims, **characterised by** troughs (6).

10. A device according to claim 8 or 9, **characterised by** small cellulose-based plates or sponges disposed if required in the troughs (6) as supports for pharmaceutical active substances and/or diagnostic agents.

11. A device according to claim 8 or 9, **characterised by** transmucosal and/or nasal therapeutic reservoir or matrix systems disposed in the troughs (6) as supports for pharmaceutical active substances and/or diagnostic agents.

12. A device according to any one of the preceding claims, **characterised in that** a second base element (1') is connected to the base element (1) via a connecting means (12) so as to form a unit.

13. Use of the device according to any one of the preceding claims to improve nasal breathing and/or for the application of pharmaceutical active substances and/or for the exposition of diagnostic agents in the nose.

## Revendications

1. Dispositif à introduire dans le nez d'un être humain, comprenant un élément de base (1, 1') du genre d'une plaque, déformable élastiquement, pouvant se déformer en forme de U le long d'un axe et pouvant être inséré, à l'état déformé en forme de U, entre la paroi de la cloison nasale et les ailes du nez, l'élément de base (1, 1') pouvant exercer une force d'écartement orientée, d'une part, sur la cloison nasale et, d'autre part, sur les ailes de nez, **caractérisé en ce que** l'élément de base (1, 1'), à l'état non chargé, inséré à l'intérieur du nez, est plan ou légèrement incurvé.

2. Dispositif selon la revendication 1, **caractérisé en ce que** l'élément de base est légèrement en forme de papillon, avec deux ailes disposées symétriquement par rapport à un axe.

3. Dispositif selon la revendication 1 ou 2, **caractérisé en ce que** l'élément de base est muni latéralement de chanfreins.

4. Dispositif selon l'une des revendications précédentes, **caractérisé en ce que** l'élément de base présente un épaississement (3) dans sa zone centrale.

5. Dispositif selon l'une des revendications précédentes, **caractérisé en ce que** l'élément de base présente un prolongement (2) du type d'un fil ou d'une barre, pour l'insertion et l'enlèvement.

6. Dispositif selon l'une des revendications précédentes, **caractérisé en ce qu'**il est formé d'un matériau convenant pour rester dans une cavité corporelle sur une longue durée.

7. Dispositif selon la revendication 6, **caractérisé en ce qu'**il est formé de caoutchouc au silicone.

8. Dispositif selon l'une des revendications précédentes, **caractérisé en ce que** l'élément de base (1, 1') est muni d'au moins un principe actif pharmaceutique à appliquer et/ou d'au moins un diagnosticum.

9. Dispositif selon l'une des revendications précédentes, **caractérisé par** des auges ou alvéoles (6).

10. Dispositif selon l'une des revendications 8 ou 9, **caractérisé par** des plaquettes ou des éponges, à base de cellulose, faisant office de supports pour des principes actifs et/ou des diagnostiques pharmaceutiques, disposés le cas échéant dans les auges ou alvéoles (6).

11. Dispositif selon l'une des revendications 8 ou 9 précédentes, **caractérisé par** des systèmes à réservoir ou à matrice de thérapie trans-mucosale et/ou nasale, disposés dans les auges ou alvéoles (6), faisant office de supports pour des principes actifs et/ou des diagnostiques pharmaceutiques.

12. Dispositif selon l'une des revendications précédentes, **caractérisé en ce qu'**un deuxième élément de base (1') est relié à l'élément de base (1) par l'intermédiaire d'un moyen de liaison (12), en formant un ensemble.

13. Dispositif selon l'une des revendications précédentes, pour l'amélioration de la respiration nasale et/ou pour l'application de principes actifs pharmaceutiques et/ou pour l'exposition de diagnostics dans le nez.
